# FASCICULE DE BREVET EUROPEEN

(11) **EP 4 182 082 B1**
(45) Date de publication et mention de la délivrance du brevet: **20.05.2026**
(21) Numéro de dépôt: 21742829.1
(22) Date de dépôt: 13.07.2021
(51) Int. Cl.: B01L 3/00

(54) **SYSTÈME DE RÉCIPIENT POUR RECEVOIR UN ÉCHANTILLON DE LIQUIDE**
BEHÄLTERSYSTEM ZUR AUFNAHME EINER FLÜSSIGKEITSPROBE
CONTAINER SYSTEM FOR RECEIVING A LIQUID SAMPLE

(30) Priorité: 15.07.2020 EP 20186086; 27.10.2020 EP 20204123
(43) Date de publication de la demande: 24.05.2023
(73) Titulaire: Gesval s.a., 4031 Angleur (BE); Université de Liège, 4000 Liège (BE)
(72) Inventeur: BUREAU, Fabrice, 6043 Ransart (BE); BUREAU, Clément, 6043 Ransart (BE); GILLET, Laurent, 6970 Tenneville (BE); VERLEYEN, Michaël, 4000 Liège (BE)
(74) Mandataire: Ipsilon Belgium
(86) Numéro de dépôt international: PCT/EP2021/069494
(87) Numéro de publication internationale: WO 2022/013235

(56) Documents cités:
- US-A- 5 277 873
- US-A1- 2011 104 737
- US-A1- 2019 250 075
- US-B2- 9 804 095

## Description

### Domaine technique de l'invention

La présente invention se rapporte au domaine de systèmes de récipient pour recevoir un échantillon de liquide.

### Etat de la technique

La pandémie actuelle engendrée par le virus SARS-CoV-2 a fait apparaître le besoin de réaliser des tests de diagnostic de manière relativement rapide et généralisée. Il s'est révélé indispensable de pouvoir tester un grand nombre de personnes en des temps très courts en vue de prendre les mesures de précautions nécessaires pour éviter une propagation rapide et exponentielle de la maladie. Les tests de diagnostic utilisés à ce jour pour détecter le virus SARS-CoV-2 nécessitent habituellement une prise d'échantillon naso-pharyngé au moyen d'un écouvillon. Cette prise est contraignante car elle nécessite un personnel qualifié pour la réaliser.

Un échantillon relativement facile à prélever est la salive. Il existe des systèmes de prélèvement de salive en vue par exemple de réaliser des analyses génétiques. Il a été démontré qu'un échantillon de salive peut aussi suffire pour la détection d'un agent pathogène, tel que le SARS-CoV-2, lors d'un test diagnostic.

Cependant, un problème qui se pose est le contrôle de la quantité d'échantillon récoltée. Afin de pouvoir effectuer un test diagnostic sur l'échantillon de liquide, et de salive en particulier, la quantité d'échantillon récoltée doit être compatible avec le volume prédéfini de liquide d'inactivation qui sera mélangé à l'échantillon afin d'assurer le transport et l'analyse de l'échantillon en toute sécurité. La quantité d'échantillon récoltée, par exemple de salive, doit être suffisante pour assurer la détection de l'agent pathogène lors du test de diagnostic mais ne doit pas être trop importante pour assurer l'inactivation de l'agent pathogène par un volume prédéfini de liquide d'inactivation. Le document US 9,804,095 B2 concerne un dispositif de mesure permettant de mettre en contact un échantillon avec un réactif. Les volumes de réactifs visés sont de l'ordre du microlitre. Ce dispositif ne permet pas un prélèvement aisé d'échantillon de liquide par un utilisateur. Le document US 5,277,873 concerne un dispositif pour mesurer un volume d'échantillon étant introduit par un capillaire dans une partie tubulaire. Ce dispositif n'est pas non plus adapté pour faciliter un prélèvement d'échantillon tel que de la salive. Les systèmes de prélèvement de salive existants font généralement appel à des entonnoirs gradués ou à des entonnoirs fixés sur des tubes gradués, mais ne permettent pas d'empêcher un remplissage trop important du tube par le donneur d'échantillon, ce qui peut empêcher la réalisation d'un test diagnostic fiable et/ou poser des risques lors du transport ou transfert de l'échantillon.

La présente invention vise donc à répondre au moins partiellement à un ou plusieurs inconvénients mentionnés ci-dessus. En particulier, l'objectif de l'invention est de fournir un système de récipient pour recevoir un échantillon de liquide qui permet un prélèvement relativement aisé et sécurisé tout en permettant un contrôle de la quantité d'échantillon.

### Résumé de l' invention

A cet effet, un premier aspect de la présente invention vise un système de récipient pour recevoir un échantillon de liquide caractérisé par les éléments cités dans la revendication 1. En particulier, le système de récipient pour recevoir un échantillon de liquide comprend un réceptacle qui inclut une partie sensiblement tubulaire adaptée à recevoir un volume prédéfini de l'échantillon. Cette partie sensiblement tubulaire a une première extrémité ouverte et une deuxième extrémité au moins partiellement ouvrable. Le réceptacle inclut également une chambre entourant au moins partiellement la partie sensiblement tubulaire, la chambre ayant une partie supérieure au moins partiellement ouverte et un fond fermé. D'une manière inventive, la partie sensiblement tubulaire traverse le fond ou s'étend à travers le fond fermé de la chambre de façon à ce que la première extrémité de la partie tubulaire s'élève à une distance du fond fermé de la chambre, par exemple à une distance entre environ 5 mm et environ 100 mm, de préférence entre environ 10 mm et environ 30 mm. Ainsi, le réceptacle est apte à recevoir un échantillon de liquide de façon relativement aisée par la première extrémité ouverte de la partie sensiblement tubulaire en quantité prédéfinie par le volume de ladite partie sensiblement tubulaire, le liquide excédant ce volume s'écoulant de la première extrémité de la partie sensiblement tubulaire vers la chambre entourant ladite partie sensiblement tubulaire qui sert de trop-plein au volume prédéfini. Comme la deuxième extrémité de la partie sensiblement tubulaire est ouvrable, l'échantillon de liquide peut être recueilli dans un autre récipient si nécessaire, par exemple dans un flacon ou fiole de laboratoire afin d'y effectuer un potentiel traitement.

Un échantillon de liquide est par exemple la salive ou le gargarisme, mais peut être étendu à tout type d'échantillon pour lequel on souhaite verser un volume défini dans un contenant. Les échantillons biologiques tels que l'urine, le sang, le plasma sont également visés, ainsi que des échantillons comme de l'eau. Un échantillon granulaire, qui a les mêmes propriétés d'écoulement que les liquides, est également considéré compris.

La deuxième extrémité de la partie tubulaire peut préférentiellement s'étendre à une distance du fond fermé de façon à ce que le fond fermé de la chambre se trouve entre la première extrémité et la deuxième extrémité de la partie tubulaire. De cette manière, l'évacuation de l'échantillon de liquide par la deuxième extrémité de la partie sensiblement tubulaire est facilitée. Alternativement, la deuxième extrémité de la partie sensiblement tubulaire pourrait se trouver à l'intérieur de la chambre entourant la partie sensiblement tubulaire.

De manière avantageuse, la partie sensiblement tubulaire peut comprendre un fond au moins partiellement en pente près de la deuxième extrémité. Une telle pente peut par exemple se terminer à la deuxième extrémité et ainsi favoriser l'écoulement de l'échantillon de liquide, en particulier en cas de liquide visqueux, vers cette deuxième extrémité. Le fond au moins partiellement en pente peut par exemple être réalisé comme un couloir de passage tel un toboggan, ou autrement.

La deuxième extrémité de la partie sensiblement tubulaire peut préférentiellement comprendre un orifice qui s'étend de préférence sur au plus une moitié d'une section transversale de la partie sensiblement tubulaire, en particulier sur au plus un tiers d'une section transversale de la partie sensiblement tubulaire. Cet orifice est configuré pour permettre une évacuation de l'échantillon de liquide qui se trouve dans le volume définie par la partie sensiblement tubulaire du réceptacle sans que le contenu éventuel de la chambre du réceptacle puisse s'évacuer.

De façon alternative, la deuxième extrémité au moins partiellement ouvrable de la partie sensiblement tubulaire peut comprendre une valve unidirectionnelle. Une telle valve, aussi appelée valve anti-retour, peut permettre un écoulement de fluide dans un sens et empêcher un passage de fluide dans le sens contraire. Dans ce cas, une valve unidirectionnelle peut par exemple être ouvrable par pression. La valve peut être apte à retenir un volume prédéfini d'échantillon de liquide quand la pression dans ledit volume est en dessous d'un seuil prédéfini et à permettre un écoulement de l'échantillon de liquide reçu quand ledit seuil prédéfini de pression est dépassé.

La partie supérieure de la chambre s'élève au-delà de la première extrémité de la partie sensiblement tubulaire, ce qui peut augmenter le volume de la chambre qui sert de trop-plein. Une chambre relativement grande peut aussi faciliter l'utilisation du réceptacle, en particulier lorsque l'échantillon est un gargarisme. Le réceptacle comprend en outre une tranchée de connexion qui relie sensiblement radialement au moins une partie de la partie supérieure ouverte de la chambre à la première extrémité de la partie sensiblement tubulaire. Cette tranchée de connexion peut par exemple ressembler à un toboggan et peut être configurée pour diriger l'échantillon de liquide vers la première extrémité de la partie sensiblement tubulaire. De préférence, le réceptacle, en particulier le bord supérieur de la chambre, peut comprendre une embouchure qui est de préférence reliée à cette tranchée de connexion. Ladite embouchure peut plus ou moins avoir la largeur d'une bouche humaine. La tranchée de connexion peut par exemple se rétrécir vers la première extrémité de la partie sensiblement tubulaire du réceptacle.

De préférence, la chambre du réceptacle peut être reliée à la partie sensiblement tubulaire du réceptacle de façon séparable. La chambre peut par exemple se visser ou se fixer sur la partie sensiblement tubulaire par serrage. De cette façon, le trop-plein peut être écarté du système de récipient relativement facilement après usage. Alternativement, le réceptacle est formé d'une seule pièce.

De manière avantageuse, la première extrémité ouverte de la partie sensiblement tubulaire du réceptacle peut être configurée pour être connectée à un conteneur, par exemple une flapule ou capsule, comprenant un liquide, par exemple un liquide d'inactivation ou tout autre type de liquide, le conteneur étant de préférence fermé par un opercule perçable ou détachable. La connexion peut par exemple se faire par vissage ou autrement. La première extrémité ouverte de la partie sensiblement tubulaire peut par exemple comprendre un élément de perçage destiné à percer un opercule du conteneur quand le conteneur est connecté au réceptacle. En faisant passer ce liquide par la partie tubulaire du réceptacle, un effet de rinçage de cette partie tubulaire est obtenu, ce qui est d'autant plus avantageux quand l'échantillon de liquide recueilli dans le réceptacle est relativement visqueux s'écoulant relativement difficilement, comme par exemple en cas d'échantillon de salive.

De préférence, le système de récipient peut comprendre un adaptateur ayant une première extrémité destinée à être connectée, de préférence de manière séparable, au réceptacle, en particulier à la deuxième extrémité de la partie sensiblement tubulaire. Ladite première extrémité de l'adaptateur peut par exemple être configurée pour une connexion directe, un raccordement ou un couplage par vissage et comprendre une rainure intérieure correspondant à un filet extérieur à la deuxième extrémité de la partie sensiblement tubulaire, ou vice-versa. Comme l'homme du métier le sait, la connexion peut se faire autrement aussi, par exemple par cliquage, par serrage, ou autrement. Une deuxième extrémité de l'adaptateur peut par exemple être configuré pour permettre une connexion ou un couplage à un tube à essai, flacon ou fiole existant de laboratoire afin de permettre l'évacuation de l'échantillon de liquide dans un contenant de test, d'analyse, de réaction ou de préservation. Ainsi, l'adaptateur du système de récipient selon l'invention permet l'utilisation du système en combinaison avec du matériel de test, de préservation ou d'analyse existant.

L'adaptateur peut avantageusement comprendre un orifice s'étendant sur au plus une partie d'une section transversale de l'adaptateur. Cet orifice est configuré pour permettre une évacuation de l'échantillon de liquide qui se trouve dans le réceptacle, et plus précisément dans la partie sensiblement tubulaire, vers un autre contenant de test, d'analyse, de réaction ou de préservation. L'étendue dudit orifice est préférentiellement adaptée à l'étendue de l'orifice de la deuxième extrémité de la partie sensiblement tubulaire du réceptacle : l'orifice de l'adaptateur peut par exemple être plus grande que l'orifice de la deuxième extrémité de la partie sensiblement tubulaire du réceptacle pour favoriser un bon écoulement de l'échantillon de liquide.

Dans une réalisation très favorable, la connexion entre l'adaptateur et le réceptacle peut être mobile entre une première position dans laquelle l'orifice de la deuxième extrémité de la partie sensiblement tubulaire et l'orifice de l'adaptateur s'alignent ou coïncident au moins partiellement, et une deuxième position dans laquelle l'orifice de la deuxième extrémité de la partie sensiblement tubulaire et l'orifice de l'adaptateur sont décalés, et ne se recoupent donc pas. Dans cette deuxième position, l'adaptateur peut fournir une fermeture étanche à l'orifice du réceptacle, tandis que dans la première position, l'échantillon de liquide peut s'évacuer du réceptacle. Cette mobilité entre ladite première position et ladite deuxième position peut s'obtenir par exemple par un mouvement de vissage ou de dévissage, ou autrement, en fonction du type de connexion ou de couplage entre le réceptacle et l'adaptateur.

L'adaptateur peut par exemple comprendre une butée apte à arrêter le réceptacle dans la deuxième position quand le réceptacle est connecté à l'adaptateur. De cette façon, l'adaptateur peut être configuré de façon à fournir une fermeture étanche au réceptacle quand le réceptacle est connecté ou couplé à l'adaptateur fournissant ainsi une position de réception d'échantillon, et de façon à permettre un écoulement de l'échantillon de liquide seulement au moment de la déconnexion ou du découplage au moins partiel du réceptacle de l'adaptateur.

Le système de récipient peut préférentiellement comprendre un flacon qui inclut une première extrémité ouverte ou ouvrable adaptée à être connectée de manière séparable au réceptacle, de préférence à l'adaptateur, en particulier à la deuxième extrémité de l'adaptateur, et une deuxième extrémité comprenant une chambre de stockage d'échantillon. Cette deuxième extrémité du flacon est fermable, de préférence fermée. La chambre de stockage d'échantillon peut s'étendre sur une partie ou sur sensiblement l'entièreté de l'intérieur du flacon. Ce flacon peut être une fiole ou tout autre type de contenant, de préférence un contenant apte à être manipulé en laboratoire, par exemple dans des procédures de tests automatisées. Ainsi, le flacon peut recueillir l'échantillon de liquide qui a été récolté à l'aide du réceptacle et transféré dans ce flacon, par exemple via l'adaptateur du système de récipient.

De préférence, la deuxième extrémité de la partie sensiblement tubulaire du réceptacle peut être apte à être connectée à la première extrémité ouverte ou ouvrable du flacon. Dans le cas d'une connexion directe, le système peut se passer d'un adaptateur, ce qui peut faciliter la manipulation du système par l'utilisateur. La connexion peut par exemple se faire par vissage, par clipsage, par serrage ou par tout autre moyen de connexion connu. La deuxième extrémité de la partie sensiblement tubulaire du réceptacle peut par exemple comprendre un pas de vis interne qui peut être vissé sur un pas de vis externe sur le flacon. Ce pas de vis, ou tout autre moyen de connexion, se trouve préférentiellement en aval d'une éventuelle valve unidirectionnelle ou d'un autre type d'orifice d'évacuation qui forme l'extrémité au moins partiellement ouvrable de la partie sensiblement tubulaire du réceptacle.

La chambre de stockage d'échantillon du flacon est de préférence configurée à au moins partiellement être préremplie d'un liquide, en particulier d'un liquide d'inactivation. Dans ce cas, la première extrémité du flacon est de préférence fermée par un opercule perçable ou détachable, par un bouchon déconnectable ou par tout autre moyen de fermeture ouvrable. La quantité de liquide préremplie peut être choisie et adaptée en fonction du volume de la partie sensiblement tubulaire du réceptacle du système de récipient. Le liquide peut être tout type de liquide adapté pour la stabilisation, la préservation, la transformation, le transport, la récupération, la neutralisation, ou l'inactivation de l'échantillon ou d'une substance contenue dans l'échantillon. Alternativement, le flacon peut être vide avant de recueillir l'échantillon de liquide récolté, et le liquide, par exemple un liquide d'inactivation, peut être fourni autrement. Il est envisageable également de prévoir deux liquides dont un premier est prérempli dans le flacon et un autre qui peut être fourni autrement. Il peut aussi s'agir du même liquide qui est partiellement fourni dans le flacon et partiellement d'une autre manière.

Le système de récipient peut également comprendre au moins un bouchon apte à fournir une fermeture sensiblement hermétique au flacon. Le bouchon peut être apte à être connecté au flacon, de préférence à la première extrémité de l'adaptateur ou de préférence à la première extrémité ouverte de la partie sensiblement tubulaire du réceptacle. Le système de récipient peut ainsi comprendre un bouchon apte à être connecté directement à la première extrémité ouverte du flacon ou être connecté au flacon via l'adaptateur, en particulier via la première extrémité de l'adaptateur, ou via l'adaptateur et/ou le réceptacle, en particulier via la première extrémité ouverte de la partie sensiblement tubulaire du réceptacle. Le système de récipient peut par exemple aussi comprendre deux bouchons, dont un premier est destiné à être connecté à la première extrémité de l'adaptateur ou à la première extrémité ouverte de la partie sensiblement tubulaire du réceptacle, et un deuxième bouchon, de préférence un bouchon sensiblement hermétique, destiné à être connecté directement à la première extrémité ouverte ou ouvrable du flacon après déconnexion de l'adaptateur du flacon. Le bouchon sensiblement hermétique destiné à refermer le flacon après dépôt de l'échantillon peut être le même ou différent d'un bouchon initialement présent sur le flacon le cas échéant. Après avoir déconnecté le réceptacle de l'adaptateur, ou après avoir déconnecté la chambre du réceptacle de la partie sensiblement tubulaire du réceptacle, et après que l'échantillon de liquide s'est évacué dans le flacon connecté à la deuxième extrémité de l'adaptateur ou à la deuxième extrémité de la partie sensiblement tubulaire du réceptacle ou dans un autre contenant, le bouchon peut fermer le système de récipient de façon étanche, afin de permettre un transport sécurisé du flacon contenant l'échantillon de liquide à tester vers un laboratoire.

De préférence, le bouchon peut comprendre une chambre destinée à contenir un liquide, la chambre étant fermée par un opercule perçable ou détachable. Ainsi, le liquide, par exemple un liquide d'inactivation, peut s'évacuer du bouchon et se mélanger à l'échantillon de liquide qui se trouve dans le flacon ou le contenant. Le volume de la chambre du bouchon, et donc la quantité de liquide, peut être choisi et adapté en fonction du volume de la partie sensiblement tubulaire du réceptacle du système de récipient. Le liquide peut être tout type de liquide adapté pour la stabilisation, la préservation, la transformation, le transport, la récupération, la neutralisation, ou l'inactivation de l'échantillon ou d'une substance contenue dans l'échantillon. L'opercule peut être percé manuellement. Alternativement, et préférentiellement, l'adaptateur, ou la partie sensiblement tubulaire du réceptacle, peut comprendre un élément de perçage destiné à percer l'opercule du bouchon quand le bouchon est connecté à l'adaptateur, ou respectivement à la partie sensiblement tubulaire du réceptacle, ce qui peut faciliter et sécuriser la manipulation du système de récipient. Comme mentionné précédemment, le liquide, par exemple un liquide d'inactivation, peut aussi être fourni dans le flacon, ou peut être fourni partiellement dans le flacon et partiellement dans le bouchon.

Le bouchon peut de préférence être apte à être inséré au moins partiellement dans la première extrémité ouverte de la partie sensiblement tubulaire du réceptacle. Le bouchon peut ainsi fournir une fermeture non seulement de forme mais aussi de force. En particulier, en augmentant les surfaces de contact entre le bouchon et la première extrémité ouverte de la partie sensiblement tubulaire du réceptacle, et en augmentant donc les forces de frottement entre ces surfaces de contact, le bouchon peut assurer une fermeture sensiblement hermétique du système de récipient de façon à ce que le système peut être transporté à un laboratoire et être manipulé dans des conditions relativement sécurisées.

De façon très avantageuse, le bouchon peut être apte, à l'insertion du bouchon dans la première extrémité ouverte de la partie sensiblement tubulaire du réceptacle, à provoquer un écoulement du volume prédéfini de l'échantillon de liquide reçu via la deuxième extrémité au moins partiellement ouvrable de la partie sensiblement tubulaire du réceptacle. Le bouchon peut ainsi exercer une double fonction : en plus de fournir une fermeture sensiblement hermétique au système de récipient, le bouchon peut augmenter la pression dans le volume qui a reçu l'échantillon de liquide et ainsi pousser ledit échantillon, comme un piston, à travers la deuxième extrémité de la partie sensiblement tubulaire du réceptacle, par exemple à travers une valve unidirectionnelle ou via tout autre orifice d'évacuation , vers un flacon connecté à la deuxième extrémité de la partie sensiblement tubulaire du réceptacle ou à la deuxième extrémité de l'adaptateur, ou vers tout autre récipient. Ainsi, la manipulation du système peut être facilitée.

Le bouchon peut préférentiellement comprendre un élément de retenue configuré pour retenir l'adaptateur de façon à permettre de découpler simultanément le bouchon et l'adaptateur du flacon, ce qui peut faciliter et accélérer la manipulation par un utilisateur ou dans un procédé automatisé, par exemple au sein d' un laboratoire. Ce découplage du bouchon et de l'adaptateur peut se faire manuellement ou machinalement, par exemple par dévissage, ou autrement selon le type de connexion utilisée. Si le bouchon est apte à être connecté à la première extrémité ouverte de la partie sensiblement tubulaire du réceptacle, la deuxième extrémité de la partie sensiblement tubulaire du réceptacle peut préférentiellement comprendre un élément de retenue configuré pour retenir le réceptacle de façon à permettre de découpler simultanément le bouchon, le réceptacle et l'adaptateur du flacon, fournissant ainsi le même avantage mentionné ci-dessus.

Un deuxième aspect de l'invention vise un procédé de prélèvement d'un échantillon de liquide caractérisé par les éléments cités dans la revendication 10. Ce procédé peut fournir un ou plusieurs avantages cités ci-dessus.

### Brève description des dessins

Un mode de réalisation préféré de l'invention sera décrit en référence aux dessins annexes dans lesquels
- les figures 1A et 1B représentent une vue de face et une vue de côté respectivement d'un système de récipient selon un mode de réalisation préféré de la présente invention ;
- les figures 2A et 2B représentent une vue en perspective du système de récipient des figures 1A et 1B pendant et après un prélèvement d'échantillon de liquide ;
- la figure 3 représente une vue en perspective du bouchon du système de récipient de la Figure 1A ;
- les figures 4A et 4B représentent des vues en coupe du réceptacle et de l'adaptateur du système de récipient de la figure 2A ;
- la figure 5 représente une vue en perspective de l'adaptateur du système de récipient de la figure 1A ;
- la figure 6 représente une vue en coupe d'un réceptacle d'un système de récipient selon un deuxième mode de réalisation préféré de la présente invention ;
- les figures 7A, 7B, 7C, 7D et 7E représentent des vues en coupe du système de récipient selon ce deuxième mode de réalisation ;
- les figures 8A, 8B et 8C représentent des vues en coupe schématiques d'un système de récipient selon un mode de réalisation alternatif.

### Description détaillée de l'invention

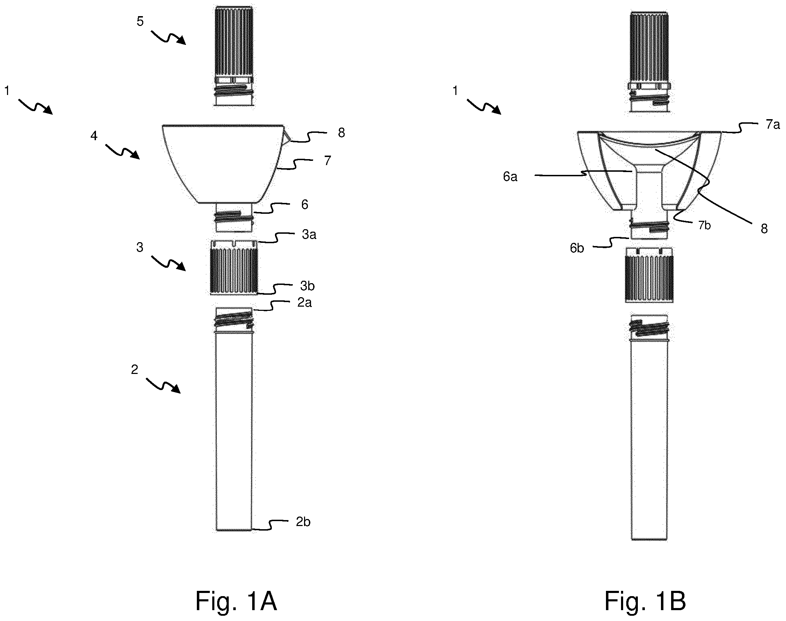

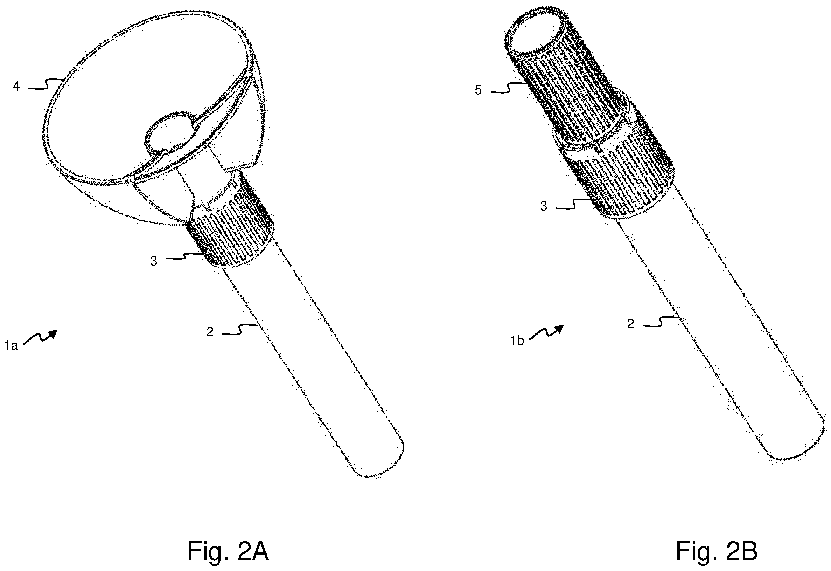

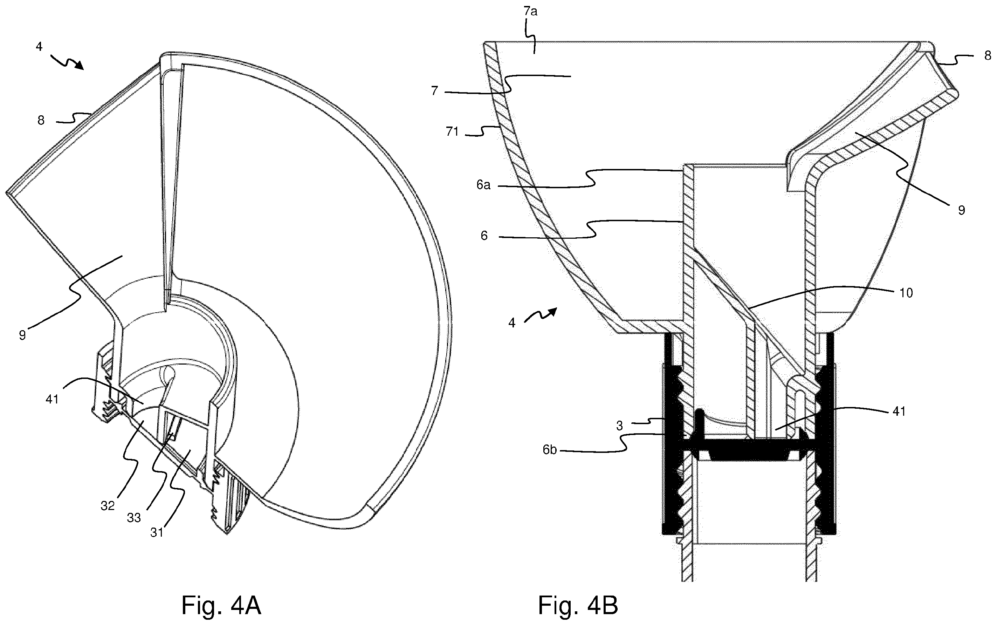

Les figures 1A et 1B représentent une vue de face et une vue de côté respectivement d'un système de récipient 1 selon un mode de réalisation préféré de la présente invention. Ce système de récipient 1 peut comprendre quatre pièces séparées : un flacon 2, un adaptateur 3, un réceptacle 4 et un bouchon 5. Le flacon 2 peut être un flacon standard de laboratoire, un tube à essai, une éprouvette ou un flacon dédié à ce système de récipient 1. Une hauteur totale du flacon 2 peut être comprise entre environ 60 mm et environ 130 mm, de préférence entre environ 70 mm et environ 90 mm, par exemple environ 80 mm. Le volume intérieur du tube est de préférence entre 2 ml et 10 ml. Le flacon 2 comprend une première extrémité 2a ouverte qui donne accès à une chambre de stockage d'échantillon à l'intérieur du flacon 2. Le flacon 2 du système de récipient 1 peut être vide, ou peut être partiellement prérempli, par exemple par un liquide d'inactivation, ou de transport ou tout autre type de liquide. Dans ce cas, la première extrémité 2a peut être muni d'un élément de fermeture ouvrable, par exemple d'un opercule perçable ou détachable ou d'un bouchon détachable, par exemple par vissage. La deuxième extrémité 2b est de préférence sensiblement conique à l'intérieur, ce qui est d'usage pour certains flacons destinés à des tests diagnostiques, mais peut par exemple avoir une forme sensiblement cylindrique ou plate à l'extérieur. La première extrémité 2a peut être munie d'un filet à l'extérieur pour permettre un vissage aisé à l'adaptateur 3, en particulier à la deuxième extrémité 3b de l'adaptateur 3. D'autres moyens de fixation détachable ou séparable sont possibles. La première extrémité 3a de l'adaptateur est configurée pour recevoir soit le réceptacle 4, soit le bouchon 5. Dans ce mode réalisation, le bouchon 5 ne se fixe pas sur le réceptacle 4, mais sur l'adaptateur 3, tel que sera illustré dans la figure 2B. Dans d'autres modes de réalisation, le bouchon 5 peut être configuré pour se fixer sur le flacon 2 directement ou sur le réceptacle 4. Le flacon 2, l'adaptateur 3 et le bouchon 5 ont de préférence une forme sensiblement cylindrique, même si d'autres formes sont envisageables. Le réceptacle 4 comprend une partie sensiblement tubulaire 6 et une chambre 7 entourant au moins partiellement cette partie sensiblement tubulaire 6. Cette chambre 7 comprend par exemple une partie évasée qui peut être interrompue par une embouchure 8. La chambre 7 a une partie supérieure 7a au moins partiellement ouverte, de préférence entièrement ouverte. La partie sensiblement tubulaire 6 a une première extrémité ouverte 6a et une deuxième extrémité 6b au moins partiellement ouvrable. La partie sensiblement tubulaire 6 traverse ou s'étend à travers le fond fermé 7b de la chambre 7 de façon à ce que la première extrémité 6a de la partie tubulaire 6 s'élève à une distance du fond fermé 7b de la chambre 7. La deuxième extrémité 6b de la partie sensiblement tubulaire 6 s'étend au-delà du fond fermé 7b de la chambre 7, de façon à ce que le fond fermé 7b de la chambre 7 se trouve entre la première extrémité 6a et la deuxième extrémité 6b de la partie tubulaire 6. Même si le réceptacle peut ressembler à un entonnoir, ce n'est pas un entonnoir, car le liquide qui est reçu dans la chambre 7 entourant au moins partiellement la partie sensiblement tubulaire 6 ne peut s'évacuer, contrairement au fonctionnement d'un entonnoir. Seulement le volume de l'échantillon de liquide reçu dans la partie sensiblement tubulaire 6 pourra être évacué. Le réceptacle 4 sera décrit après en plus de détail en référence à la figure 4.

Les figures 2A et 2B représentent une vue en perspective du système de récipient des figures 1A et 1B pendant et après un prélèvement d'échantillon de liquide. Une première étape d'un procédé de prélèvement à l'aide du système de récipient selon ce mode de réalisation préféré consiste à monter les pièces du système. Les pièces sont de préférence en matière plastique et/ou caoutchouc. Les plastiques utilisés peuvent être du PE, PP, PUR, PS, PET ainsi que tout autre plastique adapté pour cette utilisation ou toute combinaison de deux ou plusieurs de ces plastiques. Les matériaux sont de préférence configurés à résister à une température supérieure à 85°C pour les besoins de la désinfection. Un premier montage de prélèvement 1a comprend le réceptacle 4 qui est connecté à une première extrémité 3a de l'adaptateur 3 et le flacon 2 qui est connecté directement ou fixé de façon séparable à la deuxième extrémité 3b de l'adaptateur 3. Ces connexions se font de préférence par vissage, mais d'autres connexions de préférence directes sont envisageables également. Ensuite, l'utilisateur peut déposer l'échantillon de liquide dans le réceptacle 4. L'échantillon de liquide est de préférence de la salive ou un gargarisme, mais d'autres types d'échantillons de liquide biologique ou non biologique, comme l'urine, le sang, le plasma, de l'eau, ou autre peuvent être recueillis dans le réceptacle 4. Même si le système de récipient est particulièrement avantageux pour le prélèvement d'un échantillon de salive en vue de poser un diagnostic de présence d'agent pathogène, il peut également être utilisé dans toute autre application où un échantillon de volume prédéfini doit être prélevé, tel que par exemple la détection de drogue, de médicament ou de polluant. Afin de guider l'échantillon de liquide vers la partie sensiblement tubulaire 6 du réceptacle 4, le réceptacle 4 comprend une tranchée de connexion 9, tel un toboggan, qui relie au moins une partie de la partie supérieure 7a ouverte de la chambre 7 à la première extrémité 6a de la partie sensiblement tubulaire 6. Cette tranchée de connexion 9 peut comprendre une embouchure 8 à son extrémité supérieure afin de faciliter un dépôt d'échantillon de liquide tel que la salive, un liquide de crachats expectorés ou un gargarisme. La partie sensiblement tubulaire 6 est adaptée à recevoir un volume prédéfini de l'échantillon de liquide, qui va dépendre du test à réaliser sur l'échantillon. Selon les protocoles de tests de détection du virus SARS-CoV-2, un volume d'échantillon entre environ 0.1 ml et environ 5ml est souhaité, de préférence entre environ 1 ml et 2 ml. La partie sensiblement tubulaire 6 peut comprendre des graduations visibles pour donner une indication du volume recueilli à l'utilisateur. Pour éviter un dépôt d'échantillon de liquide trop grand, le réceptacle 4 est configuré de façon à fournir une zone de trop-plein, i.e. la chambre 7 entourant au moins partiellement la partie sensiblement tubulaire 6. Cette chambre a un fond fermé 7b, qui est configuré pour recevoir le volume de liquide qui déborde par-dessus de la première extrémité 6a de la partie sensiblement tubulaire 6 du réceptacle 4. Le liquide recueilli dans cette chambre 7 ne passera pas dans le flacon par après. De cette façon, il est relativement facile de contrôler la quantité d'échantillon de liquide à fournir : il suffit de remplir la partie sensiblement tubulaire 6 du réceptacle 4 jusqu'à ce que le liquide déborde, et ainsi, le volume désiré de l'échantillon de liquide peut être fourni. L'étape suivante du procédé de prélèvement d'échantillon consiste en le transfert de l'échantillon de liquide recueilli dans le réceptacle 4 vers le flacon 2. Le réceptacle 4, et en particulier la partie sensiblement tubulaire 6 du réceptacle 4, et l'adaptateur 3 ont été conçus de façon à permettre un écoulement de l'échantillon de liquide au moment de la déconnexion au moins partielle du réceptacle 4 de l'adaptateur 3. Le réceptacle 4 peut de préférence être dévissé de l'adaptateur 3. Au moment du dévissage au moins partiel du réceptacle, l'échantillon de liquide passe par l'adaptateur 3 vers le flacon 2. Le réceptacle 4 peut ensuite être jeté ou être lavé et désinfecté pour un éventuel usage suivant. Dans une étape suivante, le bouchon 5 peut être connecté à, de préférence vissé sur, la première extrémité 3a de l'adaptateur 3, obtenant ainsi un deuxième montage 1b du système de récipient. Le bouchon 5 est configuré pour fournir une fermeture sensiblement étanche au flacon 2 lorsque le bouchon 5 est connecté à l'adaptateur 3. Additionnellement, ou alternativement, le système de récipient 1 peut comprendre un bouchon sensiblement hermétique apte à être connecté au flacon 2 directement sans l'intermédiaire de l'adaptateur 3. Ce bouchon sensiblement hermétique peut être monté sur le flacon 2 après avoir déconnecté l'adaptateur 3 du flacon 2. Ce bouchon sensiblement hermétique peut optionnellement être déjà fourni de manière détachable sur le flacon 2, par exemple lorsque le flacon 2 est prérempli d'un liquide. Comme le bouchon 5, ou tout autre bouchon sensiblement hermétique, peut assurer la fermeture du flacon 2, il est souhaitable de secouer ce deuxième montage 1b du système de récipient. Ce deuxième montage 1b peut maintenant être transporté en toute sécurité à un laboratoire ou centre de test, où il est manipulé, par exemple de façon automatisée, pour y effectuer un traitement ou un test, par exemple un test diagnostique apte à détecter une charge virale, sur l'échantillon de liquide.

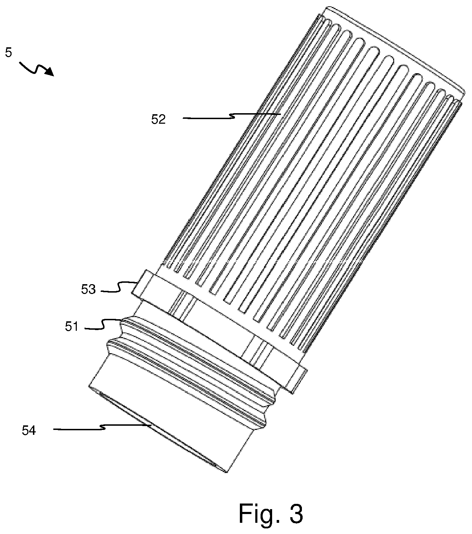

La figure 3 représente une vue en perspective du bouchon du système de récipient de la Figure 1A. La paroi latérale du bouchon 5 peut comprendre un filet 51 qui permet le vissage et dévissage du bouchon 5 sur l'adaptateur 3, ou dans un autre mode de réalisation, sur le réceptacle 4, en particulier sur la première extrémité 6a de la partie sensiblement tubulaire 6. Tout autre mode de couplage ou de connexion est également envisageable. La paroi latérale du bouchon 5 peut aussi comprendre des cannelures longitudinales 52 aptes à faciliter la prise sur le bouchon 5 lors du vissage ou dévissage du bouchon 5. Afin de faciliter et accélérer la manipulation du système de récipient manuellement ou au centre de traitement ou de test, le découplage, de préférence le dévissage, du bouchon 5 peut entraîner également le dévissage de l'adaptateur 3. A cet effet, le bouchon 5 peut comprendre un ou plusieurs éléments de retenue 53, par exemple des moulures en dent de scie sur une circonférence de la paroi latérale du bouchon 5, qui sont configurées pour agripper des éléments correspondants de l'adaptateur 3 et qui sont configurées pour retenir l'adaptateur 3 lors d'un mouvement de dévissage du bouchon 5. Le bouchon 5 peut comprendre une chambre destinée à contenir un liquide, la chambre étant fermée par un opercule perçable 54. Le liquide peut être tout type de liquide adapté pour la stabilisation, la préservation, la transformation, le transport, la récupération, la neutralisation, ou l'inactivation de l'échantillon ou d'une substance contenue dans l'échantillon. Un exemple de liquide d'inactivation est le thiocyanate de guanidium. D'autres liquides connus par l'homme du métier sont envisageables également. L'opercule 54 est configuré pour assurer une étanchéité du bouchon 5 lorsque le bouchon contient un liquide. De préférence, cet opercule est composé d'un film en plastique ou en aluminium ou un mélange d'aluminium et de plastique. L'opercule peut par exemple être collé à chaud sur le bouchon. Le volume de liquide d'inactivation est de préférence sensiblement en adéquation avec le volume de l'échantillon de liquide. En cas d'utilisation du système de récipient pour un test SARS-CoV-2, le bouchon est de préférence conçu pour pouvoir accueillir un volume de liquide entre environ 1 ml et environ 3 ml, par exemple un volume d'environ 1,5 ml du liquide par exemple d'inactivation. Après perçage de l'opercule, de préférence lors de la connexion, par exemple lors du vissage, du bouchon 5 sur l'adaptateur 3, ou alternativement lors de la connexion du bouchon 5 sur la première extrémité 6a de la partie sensiblement tubulaire 6, le liquide d'inactivation s'écoule dans le flacon 2, ce qui évite un contact de l'utilisateur avec le liquide d'inactivation. En même temps, l'écoulement du liquide via l'adaptateur 3, ou via la première extrémité 6a de la partie sensiblement tubulaire 6 du réceptacle 4 et l'adaptateur 3, permet un rinçage de ces parties, ce qui est particulièrement avantageux en cas d'échantillon relativement visqueux comme la salive. Il est également envisageable de fournir deux bouchons : un tel que décrit ci-dessus, qui peut alors être assimilé à un conteneur comprenant un liquide, tel une flapule ou une capsule, et un autre bouchon sensiblement hermétique apte à être connecté au flacon 2 directement ou via l'adaptateur 3, et configuré à fournir une étanchéité au flacon 2. Selon un mode de réalisation alternatif, le liquide d'inactivation peut aussi être fourni dans le flacon 2 du système de récipient 1 qui peut être partiellement prérempli. Après l'étape du transfert de l'échantillon de liquide recueilli dans le réceptacle 4 vers le flacon 2, l'adaptateur 3 peut être déconnecté du flacon 2 et peut être également jeté ou désinfecté. Dans une étape suivante, un bouchon sensiblement hermétique peut être connecté à, de préférence vissé sur, la première extrémité 2a du flacon 2. Il est également envisageable de combiner ces deux procédés en fournissant un flacon prérempli de liquide et un bouchon 5 comprenant un liquide. Le flacon 5 et le bouchon 2 peuvent comprendre chacun une partie d'un même liquide, ou peuvent comprendre chacun un liquide différent.

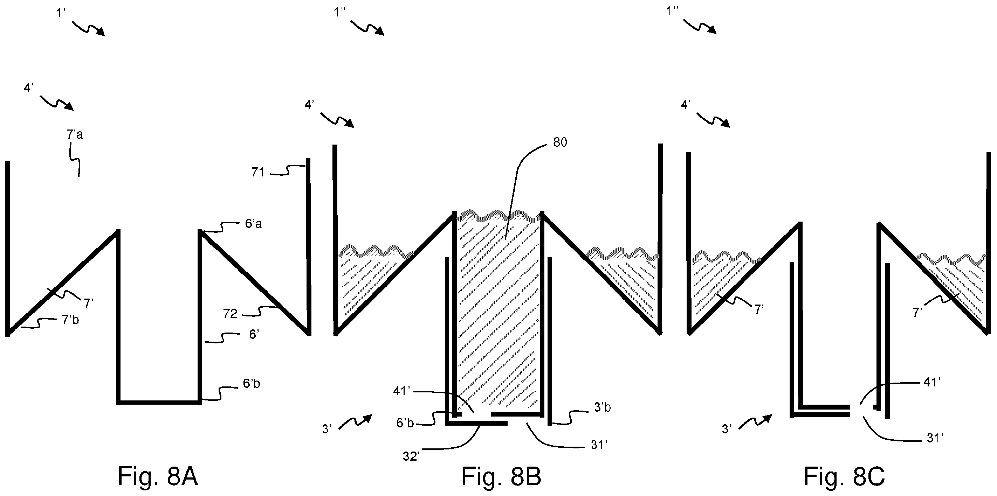

Les figures 4A et 4B représentent des vues en coupe du réceptacle et de l'adaptateur du système de récipient de la figure 2A. La partie supérieure 7a de la chambre 7 s'élève au-delà de la première extrémité 6a de la partie sensiblement tubulaire 6 du réceptacle 4. La paroi latérale 71 de la chambre 7 peut avoir une forme évasée ou au moins partiellement sensiblement conique, mais une paroi droite est également possible comme le montre le mode de réalisation illustré dans la figure 8A. Comme expliqué précédemment, le réceptacle 4 comprend une tranchée de connexion 9 qui relie de façon sensiblement radiale au moins une partie de la partie supérieure 7a ouverte de la chambre 7 à la première extrémité 6a de la partie sensiblement tubulaire 6 afin de guider l'échantillon de liquide vers l'ouverture de la partie sensiblement tubulaire 6 du réceptacle 4. La partie sensiblement tubulaire 6 comprend de préférence des couloirs ou ponts de passage afin de favoriser l'écoulement de l'échantillon de liquide, qui peut être relativement visqueux comme en cas de salive. La partie sensiblement tubulaire 6 comprend par exemple un fond 10 au moins partiellement en pente près de la deuxième extrémité 6b pour augmenter la surface de contact et favoriser l'écoulement. Le matériau sera de préférence poli et/ou verni, ou un revêtement peut être déposé sur la matière afin de favoriser l'écoulement de l'échantillon de liquide. Le réceptacle 4 de ce mode de réalisation préféré comprend un orifice 41 s'étendant sur au plus une partie d'une section transversale de l'adaptateur 3. De préférence, l'orifice 41 s'étend sur au plus un tiers de la section transversale de l'adaptateur. L'adaptateur 3 comprend également un orifice 31 s'étendant sur au plus une partie d'une section transversale de l'adaptateur 3, par exemple sur environ deux tiers de la section transversale de l'adaptateur 3. De cette façon, la section transversale de l'adaptateur 3 comprend aussi une partie fermée 32 qui s'étend sur par exemple un tiers de cette section transversale de l'adaptateur 3 et qui est apte à fournir une fermeture étanche au réceptacle 4. La connexion entre l'adaptateur 3 et le réceptacle 4 est de préférence mobile entre une première position dans laquelle l'orifice 41 de la deuxième extrémité 6b de la partie sensiblement tubulaire 6 et l'orifice 31 de l'adaptateur 3 s'alignent au moins partiellement, et une deuxième position dans laquelle l'orifice 41 de la deuxième extrémité 6b de la partie sensiblement tubulaire 6 et l'orifice 31 de l'adaptateur sont décalés, comme illustré dans la figure 4A. Ainsi, la première position fournit une communication fluidique entre le réceptacle 4 et l'adaptateur 3, tandis que la deuxième position empêche tout écoulement de l'échantillon de liquide du réceptacle 4.

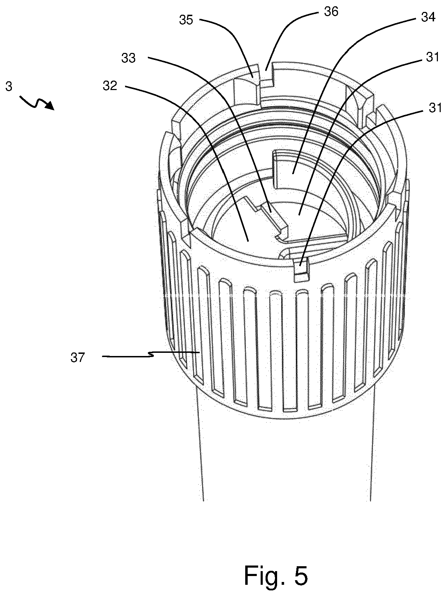

La figure 5 représente une vue en perspective de l'adaptateur du système de récipient de la figure 1A. Ce mouvement entre la première position et la deuxième position est par exemple un mouvement de rotation, par exemple un mouvement de dévissage. L'adaptateur 3 peut par exemple comprendre une butée 33 apte à arrêter le réceptacle 4 dans ladite deuxième position de fermeture quand le réceptacle 4 est connecté à l'adaptateur 3. Ainsi, après vissage du réceptacle 4 sur l'adaptateur 3, le réceptacle 4 se retrouve automatiquement dans la bonne position pour le dépôt d'un échantillon de liquide. Au moment de la déconnexion, par exemple le dévissage du réceptacle 4 de l'adaptateur 3, l'orifice 41 passera par une position d'alignement avec l'orifice 31 de l'adaptateur 3 qui établit une communication fluidique entre le réceptacle 4 et l'adaptateur 3 et permet donc un écoulement de l'échantillon de liquide vers un autre contenant, par exemple vers le flacon 2 dédié. L'adaptateur 3 peut également comprendre un élément de perçage 34 destiné à percer l'opercule 54 du bouchon 5 quand le bouchon 5 est connecté à l'adaptateur 3. Alternativement, si le bouchon 5 est configuré pour être connecté à la première extrémité ouverte 6a de la partie tubulaire 6 du réceptacle 4, la partie tubulaire 6 du réceptacle 4 peut être munie d'un tel élément de perçage. Cet élément de perçage 34 peut par exemple être une cheminée ou une paroi en arc de cercle avec un bord coupant qui s'étend au moins partiellement radialement. Cet élément de perçage 34 est de préférence relativement petit en hauteur afin de seulement percer l'opercule 54 du bouchon 5 au moment où le bouchon 5 est déjà bien connecté à l'adaptateur 3. L'adaptateur 3 peut aussi comprendre des éléments de retenue 35 qui correspondent à des éléments de retenue 53 du bouchon 5 et qui se font agripper par lesdits éléments de retenu 53 du bouchon 5 afin de permettre une déconnexion simultanée du bouchon 5 et de l'adaptateur 3. Le bord supérieur de la paroi latérale de l'adaptateur 3 peut comprendre une ou plusieurs fentes 36 configurées pour permettre un léger écartement radial de la paroi latérale lors du vissage du bouchon 5 sur l'adaptateur 3, ce qui facilite un vissage relativement souple en laissant passer les éléments de retenue 53 du bouchon sur les éléments de retenue 35 de l'adaptateur 3. La forme asymétrique de ces éléments de retenue 35 de l'adaptateur 3 permet un vissage du bouchon 5 sur l'adaptateur 3 mais entraîne le dévissage de l'adaptateur 3 lors du dévissage du bouchon 5. Comme le bouchon 5, l'adaptateur 3 peut également être muni de rainures ou cannelures longitudinales 37 sur la paroi latérale extérieure afin d'améliorer la prise sur l'adaptateur 3.

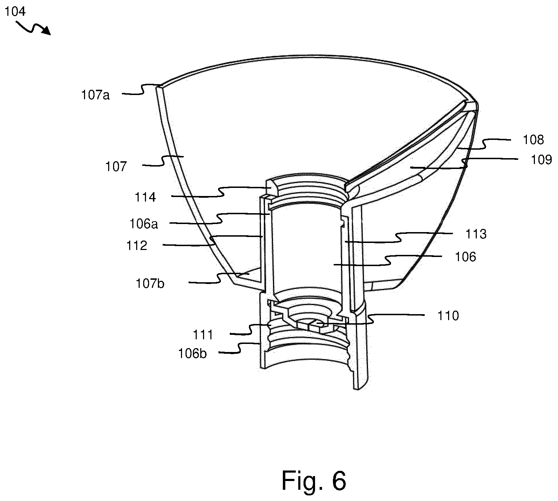

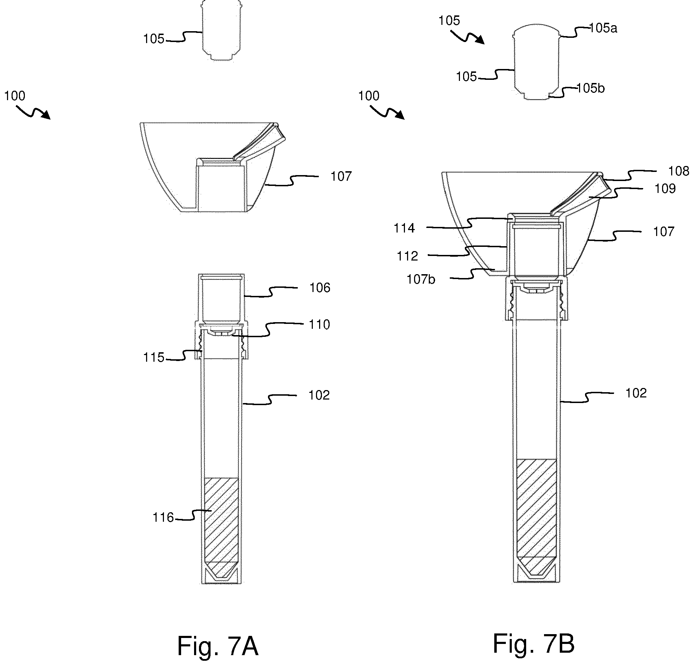

La figure 6 représente une vue en coupe d'un réceptacle 104 d'un système de récipient 100 selon un deuxième mode de réalisation préféré de la présente invention. Tout comme le réceptacle 4 de la figure 4A et 4B, le réceptacle 104 comprend une partie sensiblement tubulaire 106 et une chambre 107 entourant au moins partiellement cette partie sensiblement tubulaire 106. Cette chambre 107 comprend par exemple une partie évasée qui peut être interrompue par une embouchure 108. La chambre 107 a une partie supérieure 107a au moins partiellement ouverte, de préférence entièrement ouverte. La partie sensiblement tubulaire 106 a une première extrémité ouverte 106a et une deuxième extrémité 106b au moins partiellement ouvrable. La partie sensiblement tubulaire 106 traverse ou s'étend à travers le fond fermé 107b de la chambre 107 de façon à ce que la première extrémité 106a de la partie tubulaire 106 s'élève à une distance du fond fermé 107b de la chambre 107. La deuxième extrémité 106b de la partie sensiblement tubulaire 106 s'étend au-delà du fond fermé 107b de la chambre 107, de façon à ce que le fond fermé 107b de la chambre 107 se trouve entre la première extrémité 106a et la deuxième extrémité 106b de la partie tubulaire 106. Le réceptacle 104 comprend également une tranchée de connexion 109 qui relie de façon sensiblement radiale au moins une partie de la partie supérieure 107a ouverte de la chambre 107 à la première extrémité 106a de la partie sensiblement tubulaire 106 afin de guider l'échantillon de liquide vers l'ouverture de la partie sensiblement tubulaire 106 du réceptacle 104. Contrairement au premier mode de réalisation, la deuxième extrémité 106b au moins partiellement ouvrable de la partie sensiblement tubulaire 106 comprend une valve unidirectionnelle 110. Cette valve 110 est configurée à permettre un passage ou un écoulement d'un échantillon de liquide recueilli dans la partie sensiblement tubulaire 106 vers un autre récipient, par exemple vers un flacon 102, comme illustré dans les figures 7A à 7E. Cette valve 110 est aussi configurée pour empêcher un retour de liquide contenu dans, par exemple, un flacon connecté. La valve 110 peut se trouver à une hauteur souhaitée de la partie sensiblement tubulaire 106 en fonction du volume souhaité de la partie sensiblement tubulaire 106. Ce mode de réalisation du réceptacle 104 se différencie en plus du premier mode de réalisation en ce que la deuxième extrémité 106b de la partie sensiblement tubulaire 106 est configurée à être connectée directement à un flacon 102, en particulier à la première extrémité ouverte ou ouvrable du flacon 102. A cette fin, la deuxième extrémité 106b de la partie sensiblement tubulaire 106 peut comprendre un pas de vis interne 111 qui peut être vissé sur un pas de vis externe sur le flacon 102. Ce pas de vis, ou tout autre moyen de connexion, se trouve préférentiellement en aval d'une valve unidirectionnelle 110 ou d'un autre type d'orifice d'évacuation qui forme la deuxième extrémité 106b au moins partiellement ouvrable de la partie sensiblement tubulaire 106 du réceptacle 104. Une troisième différence avec le premier mode de réalisation est constituée par le fait que la chambre 107 peut être reliée à la partie sensiblement tubulaire 106 de façon séparable. Cette connexion peut se faire de différentes façons, par exemple par vissage, par clipsage, par serrage ou par tout autre moyen de connexion connu. La chambre 107 peut par exemple comprendre une paroi 112 sensiblement centrale et sensiblement tubulaire qui entoure une paroi latérale 113 de la partie sensiblement tubulaire 106 en la serrant. Un bord supérieur 112a de cette paroi 112 peut s'étendre radialement vers l'intérieur afin de constituer un arrêt ou une butée 114 pour la paroi 113 de la partie sensiblement tubulaire 106 quand la chambre 107 est montée sur la partie sensiblement tubulaire 106.

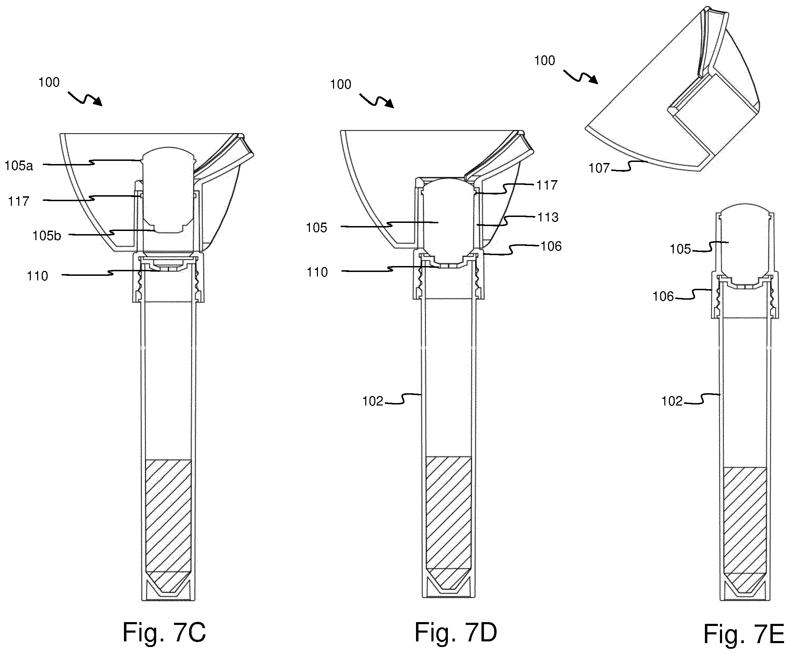

Les figures 7A, 7B, 7C, 7D et 7E représentent des vues en coupe du système de récipient 100 selon ce deuxième mode de réalisation. Le système de récipient 100 peut comprendre un flacon 102, un bouchon 105, et un réceptacle 104 incluant deux parties séparables : la chambre 107 et la partie sensiblement tubulaire 106 en mode séparé dans la Figure 7A. Le flacon 102 en soi ne diffère pas du flacon 2 du premier mode de réalisation. La première extrémité peut être munie d'un filet 115 à l'extérieur pour permettre un vissage aisé au réceptacle 104, en particulier à la deuxième extrémité 106b de la partie sensiblement tubulaire 106 du réceptacle 104. D'autres moyens de fixation détachable ou séparable sont possibles. Le flacon 102 du système de récipient 100 peut être vide, ou peut de préférence être partiellement prérempli, par exemple par un liquide d'inactivation 116, ou de transport ou tout autre type de liquide, par exemple par une quantité d'environ 1,5 ml de liquide d'inactivation ou plus ou moins selon les besoins. Afin d'éviter tout contact entre un utilisateur et ce liquide d'inactivation 116, le flacon 102 peut être muni d'une fermeture ouvrable. Dans un mode de réalisation avantageuse, le flacon 102 prérempli d'un liquide d'inactivation 116 peut être fourni à l'utilisateur avec la partie sensiblement tubulaire 106 du réceptacle 104 incluant une valve unidirectionnelle 110 connectée au, par exemple vissée sur, le flacon 102. La valve unidirectionnelle 110 peut alors fournir une fermeture sensiblement étanche au flacon 102. Il est évident pour l'homme du métier que d'autres moyens de fermeture du flacon 102 sont envisageables. Un utilisateur peut ensuite connecter la chambre 107 du réceptacle 104 à la partie sensiblement tubulaire 106 du réceptacle 104, par exemple en glissant la paroi 112 sensiblement centrale de la chambre 107 sur la partie sensiblement tubulaire 106 jusqu'à ce que la première extrémité 106a de la partie sensiblement tubulaire 106 rentre en contact avec la butée 114. Cette connexion peut également se faire autrement, par exemple par vissage, clipsage ou autre.

Le système de récipient 100, tel que montré à la figure 7B, est maintenant prêt à recevoir un échantillon de liquide. L'utilisateur peut par exemple fournir ou déposer cet échantillon en mettant l'embouchure 108 à la bouche et en crachant par exemple de la salive ou un gargarisme dans le réceptacle 104. Le liquide coulera le long de la tranchée de connexion 109 vers la partie sensiblement tubulaire 106 qui en recueillera un volume prédéfini. Un excédant de l'échantillon du liquide coulera par-dessus la première extrémité 106a de la partie sensiblement tubulaire 106 et sera recueilli dans la chambre 107, en particulier au fond fermé 107b de cette chambre 107 entourant la partie sensiblement tubulaire 106 du réceptacle 104.

Dans une étape suivante, l'échantillon de liquide reçu dans la partie sensiblement tubulaire 106 doit pouvoir s'écouler vers un autre récipient, de préférence dans le flacon 102 qui est connecté au réceptacle 104, afin de pouvoir être examiné en laboratoire. A cet effet, le bouchon 105 est configuré à être inséré dans la partie sensiblement tubulaire 106 du réceptacle 104, comme le montre la figure 7C. De manière très avantageuse, le diamètre du bouchon est choisi de façon à fournir une fermeture sensiblement étanche à la partie sensiblement tubulaire 106 du réceptacle 104. Ainsi, l'insertion du bouchon 105 dans la partie sensiblement tubulaire 106 peut augmenter la pression dans ladite partie sensiblement tubulaire 106, tel qu'un piston. Cette augmentation de pression peut alors provoquer l'ouverture de la valve unidirectionnelle 110 et causer un écoulement du volume prédéfini de l'échantillon de liquide reçu via la deuxième extrémité 106b au moins partiellement ouvrable de la partie sensiblement tubulaire 106 du réceptacle 104, en particulier via cette valve unidirectionnelle 110. Les figures 7C, 7D et 7E ne montrent pas d'échantillon de liquide ni de valve unidirectionnelle en position ouverte, mais l'homme du métier comprendra le principe. La valve unidirectionnelle 110 peut être fait en un matériau relativement souple, par exemple comprenant un élastomère, de préférence en silicone ou en caoutchouc ou tout autre matériau de type élastomère. La valve peut avoir une forme relativement pointue en V, ou une forme aplatie en U ou tout autre forme adéquate. La valve peut comprendre une seule fente, ou une pluralité de fentes, par exemple deux fentes agencées en forme de croix. La valve unidirectionnelle 110 peut être attachée, par exemple coincée ou collée ou autrement montée, à l'intérieur de la paroi intérieure 113 de la partie sensiblement tubulaire 106 du réceptacle.

Outre d'être apte à provoquer un écoulement de l'échantillon de liquide, le bouchon 105 peut également être configuré, en collaboration avec la partie sensiblement tubulaire 106 du réceptacle 104, à fournir une fermeture sensiblement étanche au flacon 102. Le bouchon 105, en particulier une première extrémité du bouchon 105, peut par exemple comprendre une flasque 105a s'étendant radialement vers l'extérieur. La partie sensiblement tubulaire 106, en particulier une première extrémité 106a, en particulier, l'intérieur de la paroi latérale 113, peut comprendre une rainure correspondante 117 apte à recevoir ladite flasque 105a du bouchon 105. Cette flasque 105a peut aider à trouver un bon emplacement du bouchon 105 en empêchant un enfoncement excessif du bouchon 105 dans la partie sensiblement tubulaire 106. Ladite flasque 105a peut en même temps améliorer l'étanchéité du flacon 102. Le bouchon 105, en particulier la deuxième extrémité 105b du bouchon 105, peut avoir une forme correspondante à la forme de la valve unidirectionnelle 110. Ainsi, après emplacement correct du bouchon 105, la deuxième extrémité 105b du bouchon 105 peut épouser la forme de la valve unidirectionnelle 110 et ainsi contribuer à la fermeture sensiblement étanche du flacon 102, comme montré à la figure 7D.

Finalement, comme le montre la figure 7E, la chambre 107 du réceptacle 104 peut être séparée de la partie sensiblement tubulaire 106 de la même façon que le montage, par exemple en glissant, en tournant, en dévissant ou par tout autre moyen de déconnexion. Ainsi, un excédant de l'échantillon de liquide recueilli dans la chambre 107 peut être jeté. Alternativement, la chambre 107 pourrait former une seule pièce avec la partie sensiblement tubulaire 106 du réceptacle 104 ou pourrait être fixée dessus de façon inséparable. Le flacon 102 peut être fermé de façon sensiblement étanche par le bouchon 105 inséré dans la partie sensiblement tubulaire 106 du réceptacle dont la deuxième partie 106b reste connectée à, par exemple vissée sur, la première partie ouverte du flacon 102. Le flacon peut ainsi être envoyé à un laboratoire de façon sécurisée. Au laboratoire, la partie sensiblement tubulaire 106 du réceptacle 104 incluant la valve unidirectionnelle 110 pourra être déconnectée, par exemple dévissée, par exemple de façon automatisée, du flacon 102 sans que cela nécessite une quelconque manipulation du bouchon 105, et l'échantillon de liquide peut subir les tests et/ou analyses souhaités.

Les figures 8A, 8B et 8C représentent des vues en coupe schématiques d'un système de récipient selon un mode de réalisation alternatif. Dans la figure 8A, le système de récipient 1' comprend uniquement un réceptacle 4' qui peut éventuellement être utilisé en combinaison avec un flacon, une fiole de laboratoire, un tube à essai ou tout autre contenant existant. Comme c'est le cas dans le mode réalisation préféré, le réceptacle 4' inclut une partie sensiblement tubulaire 6' adaptée à recevoir un volume prédéfini de l'échantillon de liquide. La partie sensiblement tubulaire 6' a une première extrémité 6'a ouverte et une deuxième extrémité 6'b au moins partiellement ouvrable. L'ouverture de la deuxième extrémité 6'b peut être réalisé par exemple par un orifice couvert par un opercule, ou par un fond prévu d'un bouchon ou d'un clapet ou par tout autre moyen connu par l'homme du métier. Cette deuxième extrémité peut par exemple avoir une forme sensiblement cylindrique comme illustré ou alternativement une forme sensiblement conique afin d'améliorer l'écoulement de l'échantillon de liquide. Le réceptacle 4' comprend également une chambre 7' entourant au moins partiellement la partie sensiblement tubulaire 6', la chambre 7' ayant une partie supérieure 7'a au moins partiellement ouverte ou entièrement ouverte et un fond fermé 7'b. La partie sensiblement tubulaire 6' s'étend à travers le fond fermé 7'b de la chambre 7 de façon à ce que la première extrémité 6'a de la partie sensiblement tubulaire 6' s'élève à une distance du fond fermé 7'b de la chambre 7', dans ce cas du point le plus bas du fond fermé 7'b. La première extrémité 6'a peut par exemple s'élever à une distance d'entre environ 5 mm à environ 100 mm du fond fermé 7'b de la chambre 7'. Dans ce mode de réalisation, la paroi latérale 71 de la chambre 7' est une paroi sensiblement droite et ne forme pas une partie évasée comme dans un entonnoir. Le fond 7'b de la chambre 7' est formé par une partie inclinée 72 entourant la partie sensiblement tubulaire 6' qui rejoint la paroi latérale 71 à une hauteur entre la première et la deuxième extrémité 6'a, 6'b de la partie sensiblement tubulaire 6' afin de former un trop-plein autour de la partie sensiblement tubulaire 6'. La partie inclinée 72 est configurée pour diriger le liquide qui déborde de la partie sensiblement tubulaire 6' vers le fond 7'b de la chambre 7 de façon à ce que la partie sensiblement tubulaire 6' contienne le volume désiré de l'échantillon de liquide.

Dans les figures 8B et 8C, le système de récipient 1" comprend un réceptacle 4' et un adaptateur 3'. Comme dans le mode de réalisation précédent illustré dans la figure 8A, le système de récipient peut éventuellement être utilisé en combinaison avec un flacon ou une fiole de laboratoire ou tout autre contenant existant. Le réceptacle 4' est semblable au mode de réalisation de la figure 8A également. La différence se situe dans le fonctionnement et l'interaction entre le réceptacle 4' et l'adaptateur 3'. Comme le montre la figure 8B, une coupe de l'adaptateur 3' montre une forme en coupe sensiblement en U ayant une première extrémité 3'a configurée pour être connectée au réceptacle, et en particulier à la deuxième extrémité 6'b de la partie sensiblement tubulaire 6' du réceptacle 4'. L'adaptateur 3', en particulier une deuxième extrémité 3'b de l'adaptateur 3', comprend un orifice 31' s'étendant sur au plus une partie d'une section transversale 30 de l'adaptateur 3'. Comme l'orifice ne s'étend que sur une partie de la section transversale, il reste une partie fermée 32 de la section transversale 30. Cette partie fermée 32' est telle qu'elle arrive à fournir une fermeture étanche au réceptacle 4' dont la deuxième extrémité 6'b de la partie sensiblement tubulaire 6 comprend également un orifice 41' qui s'étend également sur au plus une moitié d'une section transversale de la partie sensiblement tubulaire 6'. La connexion entre l'adaptateur 3' et le réceptacle 4' est de préférence mobile entre une première position illustrée dans la figure 8C dans laquelle l'orifice 41' de la deuxième extrémité de la partie sensiblement tubulaire 6' et l'orifice 31 de l'adaptateur 3' s'alignent au moins partiellement, et une deuxième position illustrée dans la figure 8B dans laquelle l'orifice 41' de la deuxième extrémité de la partie sensiblement tubulaire 6' et l'orifice 31' de l'adaptateur sont décalés, et donc ne s'alignent pas. Cette deuxième position fournit une position fermée du réceptacle 4', tandis que la première position fournit une position ouverte, par exemple en présence d'un contenant afin de transvaser un volume prédéfini de l'échantillon de liquide 80 du réceptacle vers un contenant de laboratoire sans que le liquide présent dans la chambre 7' ne s'écoule.

Même si la présente invention a été illustrée en référence à des modes de réalisation spécifiques, l'homme du métier comprendra que l'invention n'est pas limitée aux détails des modes de réalisations illustratifs, et que la présente invention peut être réalisée avec de nombreuses modifications sans s'écarter de la portée de l'invention. Les modes de réalisation doivent être considérés comme illustratifs et non de façon restrictive, la portée de l'invention étant définie par les revendications qui suivent plutôt que par la description qui précède. Toute modification qui entre dans la signification ou l'équivalence des revendications est destinée à être comprise. Autrement dit, il est envisagé de couvrir toutes les modifications, variations ou équivalences qui tombent sous la portée des principes de base sous-jacents et dont les caractéristiques essentielles sont revendiquées dans cette demande de brevet. Le lecteur de cette demande de brevet comprendra que les mots "comprenant" ou "comprend" n'excluent pas d'autre élément ou étape, et que les mots "un" ou "une" n'excluent pas une pluralité. Les signes de références dans les revendications ne peuvent être considérés comme limitant la revendication concernée. Les termes "premier", "deuxième", "troisième", "a", "b", "c", etcetera sont introduits pour distinguer différents éléments ou étapes et ne décrivent pas nécessairement un ordre séquentiel ou chronologique. De même, les termes "supérieure", "inférieure", "dessus", "dessous", etcetera sont introduits à des fins descriptives et pas nécessairement pour désigner des positions relatives. On comprendra que ces termes sont interchangeables sous des conditions appropriées et que des modes de réalisations de l'invention sont capables d'être opérables selon la présente invention dans d'autres séquences ou dans des orientations qui diffèrent de celles décrites ou illustrées ci-dessus.

## Revendications

1. Système de récipient (1, 100) pour recevoir un échantillon de liquide comprenant un réceptacle (4, 104) incluant :
- une partie sensiblement tubulaire (6, 106) adaptée à recevoir un volume prédéfini de l'échantillon, la partie sensiblement tubulaire ayant une première extrémité ouverte (6a, 106a) et une deuxième extrémité au moins partiellement ouvrable (6b, 106b) ;
- une chambre (7, 107) entourant au moins partiellement la partie sensiblement tubulaire, la chambre ayant une partie supérieure au moins partiellement ouverte (7a, 107a) et un fond fermé (7b, 107b) ;
la partie sensiblement tubulaire (6, 106) traversant le fond fermé de la chambre de façon à ce que la première extrémité de la partie sensiblement tubulaire s'élève à une distance du fond fermé de la chambre, dans lequel la partie supérieure de la chambre s'élève au-delà de la première extrémité de la partie sensiblement tubulaire, **caractérisé en ce que** le réceptacle comprend une tranchée de connexion (9, 109) qui relie de façon sensiblement radiale au moins une partie de la partie supérieure ouverte de la chambre à la première extrémité de la partie sensiblement tubulaire.

2. Système de récipient (100) selon l'une quelconque des revendications précédentes, dans lequel la chambre est reliée à la partie sensiblement tubulaire de façon séparable.

3. Système de récipient (1, 100) selon l'une quelconque des revendications précédentes, dans lequel la deuxième extrémité au moins partiellement ouvrable de la partie sensiblement tubulaire comprend une valve unidirectionnelle (110).

4. Système de récipient (1, 100) selon l'une quelconque des revendications précédentes, comprenant un flacon (2, 102) comprenant une première extrémité (2a) ouverte ou ouvrable adaptée à être connectée de manière séparable au réceptacle, et une deuxième extrémité (2b) comprenant une chambre de stockage d'échantillon.

5. Système de récipient (1, 100) selon la revendication 4, dans lequel la deuxième extrémité de la partie sensiblement tubulaire du réceptacle est apte à être connectée à la première extrémité ouverte ou ouvrable du flacon.

6. Système de récipient (1, 100) selon l'une quelconque des revendications 4 - 5, dans lequel la chambre de stockage d'échantillon du flacon est configurée pour être préremplie au moins partiellement d'un liquide, en particulier d'un liquide d'inactivation.

7. Système de récipient (1, 100) selon l'une quelconque des revendications 4 - 6, comprenant au moins un bouchon (5, 105) apte à fournir une fermeture sensiblement hermétique au flacon.

8. Système de récipient (1, 100) selon la revendication 7, dans lequel le bouchon est apte à être inséré au moins partiellement dans la première extrémité ouverte de la partie sensiblement tubulaire du réceptacle.

9. Système de récipient (1, 100) selon la revendication 8, dans lequel le bouchon est apte, à l'insertion du bouchon dans la première extrémité ouverte de la partie sensiblement tubulaire du réceptacle, à provoquer un écoulement du volume prédéfini de l'échantillon de liquide reçu via la deuxième extrémité au moins partiellement ouvrable de la partie sensiblement tubulaire du réceptacle.

10. Procédé de prélèvement d'un échantillon de liquide comprenant les étapes de
- la fourniture d'un système de récipient selon l'une quelconque des revendications précédentes ;
- optionnellement, la connexion de la chambre du réceptacle à la partie sensiblement tubulaire du réceptacle ;
- optionnellement, la connexion du réceptacle au flacon, qui est optionnellement au moins partiellement prérempli d'un liquide ;
- la fourniture de l'échantillon de liquide au réceptacle ;
- l'ouverture de la deuxième extrémité de la partie sensiblement tubulaire afin de prélever le volume prédéfini de l'échantillon de liquide, l'ouverture de la deuxième extrémité de la partie sensiblement tubulaire se faisant de préférence en mettant un bouchon sur la première extrémité ouverte de la partie sensiblement tubulaire du réceptacle ;
- optionnellement, la déconnexion de la chambre du réceptacle de la partie sensiblement tubulaire du réceptacle.

## Patentansprüche

1. Behältersystem (1, 100) zur Aufnahme einer Flüssigkeitsprobe, umfassend ein Behältnis (4, 104), das Folgendes beinhaltet:
- einen im Wesentlichen rohrförmigen Teil (6, 106), der angepasst ist, um ein vordefiniertes Volumen der Probe aufzunehmen, wobei der im Wesentlichen rohrförmige Teil ein erstes offenes Ende (6a, 106a) und ein zweites, zumindest teilweise öffnungsfähiges Ende (6b, 106b) aufweist;
- eine Kammer (7, 107), die den im Wesentlichen rohrförmigen Teil zumindest teilweise umgibt, wobei die Kammer einen zumindest teilweise offenen Teil (7a, 107a) und einen geschlossenen Boden (7b, 107b) aufweist;
wobei der im Wesentlichen rohrförmige Teil (6, 106) den geschlossenen Boden der Kammer durchquert, sodass sich das erste Ende des im Wesentlichen rohrförmigen Teils in einem Abstand von dem geschlossenen Boden der Kammer erhebt, wobei sich der obere Teil der Kammer über das erste Ende des im Wesentlichen rohrförmigen Teils hinaus erhebt, **dadurch gekennzeichnet, dass** das Behältnis einen Verbindungsgraben (9, 109) umfasst, der auf im Wesentlichen radiale Weise mindestens einen Teil des offenen oberen Teils der Kammer an das erste Ende des im Wesentlichen rohrförmigen Teils anbindet.

2. Behältersystem (100) nach einem der vorhergehenden Ansprüche, wobei die Kammer auf trennbare Weise an den im Wesentlichen rohrförmigen Teil angebunden ist.

3. Behältersystem (1, 100) nach einem der vorhergehenden Ansprüche, wobei das zweite, zumindest teilweise öffnungsfähige Ende des im Wesentlichen rohrförmigen Teils ein Einwegventil (110) umfasst.

4. Behältersystem (1, 100) nach einem der vorhergehenden Ansprüche, umfassend ein Fläschchen (2, 102), umfassend ein erstes offenes oder öffnungsfähiges Ende (2a), das angepasst ist, um auf trennbare Weise mit dem Behältnis verbunden zu werden, und ein zweites Ende (2b), umfassend eine Probenspeicherkammer.

5. Behältersystem (1, 100) nach Anspruch 4, wobei das zweite Ende des im Wesentlichen rohrförmigen Teils des Behältnisses geeignet ist, um mit dem ersten offenen oder öffnungsfähigen Ende des Fläschchens verbunden zu werden.

6. Behältersystem (1, 100) nach einem der Ansprüche 4-5, wobei die Probenspeicherkammer des Fläschchens konfiguriert ist, um zumindest teilweise mit einer Flüssigkeit, insbesondere einer Inaktivierungsflüssigkeit, vorgefüllt zu sein.

7. Behältersystem (1, 100) nach einem der Ansprüche 4-6, umfassend mindestens einen Stopfen (5, 105), der geeignet ist, um einen im Wesentlichen hermetischen Verschluss für das Fläschchen bereitzustellen.

8. Behältersystem (1, 100) nach Anspruch 7, wobei der Stopfen geeignet ist, um zumindest teilweise in das erste offene Ende des im Wesentlichen rohrförmigen Teils des Behältnisses eingesetzt zu werden.

9. Behältersystem (1, 100) nach Anspruch 8, wobei der Stopfen beim Einsetzen des Stopfens in das erste offene Ende des im Wesentlichen rohrförmigen Teils des Behältnisses geeignet ist, um einen Fluss des vordefinierten Volumens der Flüssigkeitsprobe zu bewirken, die über das zweite, zumindest teilweise öffnungsfähige Ende des im Wesentlichen rohrförmigen Teils des Behältnisses erhalten wird.

10. Verfahren zum Entnehmen einer Flüssigkeitsprobe, umfassend die folgenden Schritte
- Bereitstellen eines Behältersystems nach einem der vorhergehenden Ansprüche;
- optional Verbinden der Kammer des Behältnisses mit dem im Wesentlichen rohrförmigen Teil des Behältnisses;
- optional Verbinden des Behältnisses mit dem Fläschchen, das optional zumindest teilweise mit einer Flüssigkeit vorgefüllt ist;
- Bereitstellen der Flüssigkeitsprobe für das Behältnis;
- Öffnen des zweiten Endes des im Wesentlichen rohrförmigen Teils, um das vordefinierte Volumen der Flüssigkeitsprobe zu entnehmen, wobei das Öffnen des zweiten Endes des im Wesentlichen rohrförmigen Teils vorzugsweise durch Aufsetzen eines Stopfens auf das erste offene Ende des im Wesentlichen rohrförmigen Teils des Behältnisses erfolgt;
- optional Trennen der Kammer des Behältnisses von dem im Wesentlichen rohrförmigen Teil des Behältnisses.

## Claims

1. Container system (1, 100) for receiving a liquid sample comprising a receptacle (4, 104) including:
- a substantially tubular portion (6, 106) for receiving a predefined volume of the sample, the substantially tubular portion having a first open end (6a, 106a) and an at least partially openable second end (6b, 106b);
- a chamber (7, 107) at least partially surrounding the substantially tubular portion, the chamber having an at least partially open upper portion (7a, 107a) and a closed bottom (7b, 107b);
the substantially tubular portion (6, 106) extending through the closed bottom of the chamber, such that the first end of the substantially tubular portion rises at a distance from the closed bottom of the chamber, wherein the upper portion of the chamber rises beyond the first end of the substantially tubular portion, **characterised in that** the receptacle comprises a connecting trench (9, 109) which substantially radially connects at least a portion of the open upper portion of the chamber to the first end of the substantially tubular portion.

2. Container system (100) according to any one of the preceding claims, wherein the chamber is separably connected to the substantially tubular portion.

3. Container system (1, 100) according to any one of the preceding claims, wherein the at least partially openable second end of the substantially tubular portion comprises a one-way valve (110).

4. Container system (1,100) according to any one of the preceding claims, comprising a vial (2,102) comprising a first open or openable end (2a) adapted to be separably connected to the container, and a second end (2b) comprising a sample storage chamber.

5. Container system (1, 100) according to claim 4, wherein the second end of the substantially tubular portion of the receptacle is adapted to be connected to the first open or openable end of the vial.

6. Container system (1, 100) according to any one of claims 4 to 5, wherein the sample storage chamber of the vial is configured to be at least partially pre-filled with a liquid, in particular, an inactivation liquid.

7. Container system (1, 100) according to any one of claims 4 to 6, comprising at least one cap (5, 105) adapted to provide a substantially airtight closure to the vial.

8. Container system (1,100) according to claim 7, wherein the cap is capable of being inserted at least partially into the first open end of the substantially tubular portion of the receptacle.

9. Container system (1, 100) according to claim 8, wherein the cap is capable, upon insertion of the cap into the first open end of the substantially tubular portion of the receptacle, of causing a flow of the predefined volume of the received liquid sample via the at least partially openable second end of the substantially tubular portion of the receptacle.

10. Method for taking a liquid sample comprising the steps of
- providing a container system according to any one of the preceding claims;
- optionally, connecting the chamber of the receptacle to the substantially tubular portion of the receptacle;
- optionally, connecting the receptacle to the vial, which is optionally at least partially pre-filled with a liquid;
- providing the liquid sample to the receptacle;
- opening the second end of the substantially tubular portion to take the predefined volume of the liquid sample, the opening of the second end of the substantially tubular portion preferably being done by putting a cap on the first open end of the substantially tubular portion of the receptacle;
- optionally, disconnecting the chamber of the receptacle from the substantially tubular portion of the receptacle.
